# EUROPEAN PATENT SPECIFICATION

(11) **EP 2 306 968 B1**
(45) Date of publication and mention of the grant of the patent: **07.12.2016**
(21) Application number: 09769224.8
(22) Date of filing: 23.06.2009
(51) Int. Cl.: A61K 8/37, A61K 8/92, A61K 8/895, A61K 8/85, A61Q 1/00, A61Q 1/02, A61Q 1/04, A61Q 1/06, A61Q 1/10

(54) **COSMETIC COMPOSITION CONTAINING A SILICONE ACRYLATE AND A POLYESTER WAX**
EIN SILIKONACRYLAT UND EIN POLYESTERWACHS ENTHALTENDE KOSMETISCHE ZUSAMMENSETZUNG
COMPOSITION COSMÉTIQUE CONTENANT UNE SILICONE ACRYLATE ET UNE CIRE POLYESTER

(30) Priority: 25.06.2008 FR 0854207
(43) Date of publication of application: 13.04.2011
(73) Proprietor: Chanel Parfums Beauté, 92521 Neuilly sur Seine Cedex (FR)
(72) Inventor: MALVEZIN, Chantal, F-60260 Lamorlaye (FR); PIERRE, Stéphanie, F-75010 Paris (FR); CARLES, Céline, F-75009 Paris (FR)
(74) Representative: Cabinet Plasseraud
(86) International application number: PCT/EP2009/057768
(87) International publication number: WO 2009/156375

(56) References cited:
- EP-A- 0 963 751
- EP-A1- 1 785 773
- FR-A- 2 740 037
- FR-A- 2 878 738
- JP-A- 11 001 530
- US-A1- 2003 097 012
- "Mascara:Water Resistant-Formulation 00874"[Online] 27 March 2006 (2006-03-27), XP002516261 Retrieved from the Internet: URL:http://www.dowcorning.co.jp/ja_JP/cont ent/publishedlit/FORMUL_00874.pdf> [retrieved on 2009-02-18]
- "Liptissime Duo Stick: Lipstick: Comfort to wear, Long Lasting, Formulation 00840"[Online] 27 March 2006 (2006-03-27), XP002516262 Retrieved from the Internet: URL:http://www.dowcorning.co.jp/ja_JP/cont ent/publishedlit/FORMUL_00840pdf> [retrieved on 2009-02-18]
- B.RITTER ET AL.: "Detection of coating waxes on apples by differential scanning calorimetry" EUR. FOOD RES. TECHNOL., vol. 212, 2001, pages 603-607, XP002551553
- A. P. TULLOCH: "The composition of beeswax and other waxes secreted by insects" LIPIDS, vol. 5, no. 2, February 1970 (1970-02), pages 247-258, XP009124443
- ULLMANN'S ENCYCLOPEDIA OF INDUSTRIAL CHEMISTRY, vol. 39, 15 June 2000 (2000-06-15), pages 119-121,

## Description

The present invention relates to a cosmetic composition intended for caring for or making up the skin, such as a foundation, a lipstick or an eyeshadow. It also relates to a cosmetic method for caring for or making up the skin, comprising the topical application of this composition to the skin.

Dow Corning has proposed a novel silicone dendrimer grafted acrylate copolymer such as an acrylate/poly-trimethylsiloxymethacrylate copolymer. Dow Corning suggested its incorporation into lipsticks, mascaras, nail varnishes and foundations for improving colour retention, lack of transfer and water resistance while obtaining a satisfactory sheen level (EP0963751). This silicone acrylate copolymer makes it possible to improve the retention level of the compositions on the skin without reducing the feelings of ease of application and comfort over time. However, it is necessary to combine it with structuring agents in order to give the cosmetic composition a consistency compatible with its mode of application.

Among the structuring agents known in cosmetics are waxes, pasty compounds and gelling agents. It is known by a person skilled in the art who formulates cosmetic products that the incorporation of waxes into a composition in order to structure it reduces its sheen. The Applicant has found that it is possible to structure a composition containing a silicone acrylate copolymer as mentioned previously in order to obtain a film of good retention, which has a sufficient consistency for 1) being able to be applied and 2) not migrating away from the initial deposition of the composition on the skin, without being too rigidified to retain the benefit of the elasticity procured by the copolymer.

The Applicant has found that the addition of a particular polyester wax makes it possible to provide the composition containing the silicone acrylate copolymer with flexibility, elasticity, and comfort (no tugging sensation). It also makes it possible to minimize the risks of fluffing. The polyester wax used in the context of the invention is advantageously compatible with the silicone acrylate and has a certain degree of crystallinity, although the composition is stable (in the case of a fluid cosmetic composition, it does not phase separate). Without affecting the retention of the composition on the skin, and more particularly its non-transfer properties, it surprisingly makes it possible to retain a good sheen level.

A polyester wax has especially already been described in Application US 2006/0008489 as a gelling agent for oily makeup products, containing in particular branched hydrocarbons, but it has never been used, to the knowledge of the Applicant, in combination with a silicone acrylate.

One subject of the present invention is therefore a cosmetic composition for making up or caring for the skin comprising, in a physiologically acceptable medium:
(a) 0.6 to 20% by weight relative to the total weight of said composition of at least one polyester wax having a melting point between 40 and 90°C, and a degree of crystallinity below 95%; wherein the degree of crystallinity of the polyester wax at 25°C is equal to the ratio of the total amount of crystals present at a temperature of 25°C based on 200 J/g said polyester wax being chosen from:
   - a polyester of at least one polyol and of at least two, or even of at least three, identical or different, saturated or unsaturated, linear or branched, aliphatic monocarboxylic acids having, independently of one another, from 8 to 30 carbon atoms,
   - the polyester wax obtained from (i) a saturated or unsaturated, linear or branched, aliphatic monocarboxylic acid having from 8 to 30 carbon atoms; (ii) a linear or branched diacid having from 12 to 36 carbon atoms; and (iii) glycerol,
   - beeswax derivatives selected from the waxes having the INCI name Synthetic Beeswax, Bis-PEG-12 Dimethicone Beeswax, and mixtures thereof, and
   - polyester obtained by reaction i) of a diacid dimer of isostearic acid with ii) an aliphatic alcohol,
      and
(b) from 1 to 25% relative to the total weight of said composition of at least one acrylate/polytrimethylsiloxymethacrylate copolymer.

According to a preferred embodiment, said composition has rheological properties such that:
- its average elastic modulus G' is between 0.1 and 120 000 Pa, and
- its average viscous modulus G'' is between 30 and 30 000 Pa.

Thus, one subject of the present invention is a cosmetic composition for making up or caring for the skin comprising, in a physiologically acceptable medium:
(a) 0.6 to 20% by weight relative to the total weight of said composition of at least one polyester wax having a melting point between 40 and 90°C, and a degree of crystallinity below 95%; wherein the degree of crystallinity of the polyester wax at 25°C is equal to the ratio of the total amount of crystals present at a temperature of 25°C based on 200 J/g
   said polyester wax being chosen from:
   - a polyester of at least one polyol and of at least two, or even of at least three, identical or different, saturated or unsaturated, linear or branched, aliphatic monocarboxylic acids having, independently of one another, from 8 to 30 carbon atoms,
   - the polyester wax obtained from (i) a saturated or unsaturated, linear or branched, aliphatic monocarboxylic acid having from 8 to 30 carbon atoms; (ii) a linear or branched diacid having from 12 to 36 carbon atoms; and (iii) glycerol,
   - beeswax derivatives selected from the waxes having the INCI name Synthetic Beeswax, Bis-PEG-12 Dimethicone Beeswax, and mixtures thereof, and
   - polyester obtained by reaction i) of a diacid dimer of isostearic acid with ii) an aliphatic alcohol,
      and
(b) from 1 to 25% relative to the total weight of said composition of at least one acrylate/polytrimethylsiloxymethacrylate copolymer,
   said wax and said copolymer being in an amount such that said composition has, at 20°C in the ambient atmosphere:
   - an average elastic modulus G' between 0.1 and 120 000 Pa; and
   - an average viscous modulus G'' between 30 and 30 000 Pa.

### Rheological evaluation

The viscoelastic behaviour of the composition according to the invention may be evaluated on a GEMINI rheometer at 20°C in the ambient atmosphere, the device being used in dynamic mode with a set strain (1%) and equipped with an acrylic plate-plate geometry with a gap of 1000 µm. The upper plate was driven by an oscillating movement about its axis with a stress sweep σ of 0.01 to 1000 Pa at a pulsation of 1 Hz.

The average modulus G' in the sense of the present invention is the elastic modulus in the linear area of the curve G'(σ). The modulus G' measured for the compositions of the invention is preferably between 0.1 and 120 000 Pa, preferably between 100 and 50 000 Pa, more preferably between 700 and 35 000 Pa.

The average modulus G'' in the sense of the present invention is the viscous modulus in the linear area of the curve G''(σ). The modulus G'' measured for the compositions of the invention is preferably between 30 and 30 000 Pa, preferably between 100 and 10 000 Pa, more preferably between 300 and 5000 Pa.

### Acrylate/polytrimethylsiloxymethacrylate copolymer

The acrylate/polytrimethylsiloxymethacrylate copolymer comprises a carbosiloxane dendrimer structure grafted onto a vinyl backbone. The expression "carbosiloxane dendrimer structure" denotes a branched structure provided with high-molecular-weight groups that are branched with high regularity in the radial direction starting from the backbone. These structures are described in the Japanese Patent Application Kokai 9-171 154.

The acrylate/polytrimethylsiloxymethacrylate copolymer may be defined more generally as a vinyl polymer containing a carbosiloxane dendrimer represented by the following general formula: 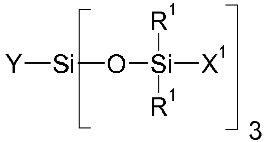 in which R¹ represents an aryl group or an alkyl group containing from 1 to 10 carbon atoms, and X¹ represents a silylalkyl group represented by the formula: 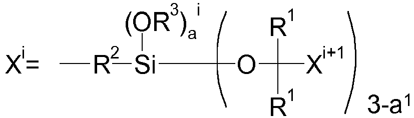 in which:
R¹ is defined above;
R² represents an alkylene group containing 2 to 10 carbon atoms;
R³ represents an alkyl group containing 1 to 10 carbon atoms;
Xⁱ⁺¹ represents a hydrogen atom, an alkyl group containing 1 to 10 carbon atoms, an aryl or silylalkyl group defined above with i = i + 1;
i is an integer from 1 to 10 which represents the iteration of said silylalkyl;
Y represents an organic group that may be a (meth)acrylic or (meth)acrylamide group, and which is preferably represented by the formulae: 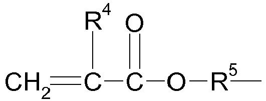 and 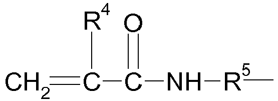 in which R⁴ represents a hydrogen atom or an alkyl group, such as a methyl group, R⁵ represents an alkylene group containing from 1 to 10 carbon atoms, such as a methylene, ethylene, propylene or butylene group, or an organic group that contains a styryl group and which is represented by the formula: 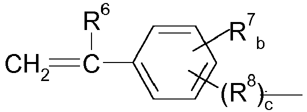 in which R⁶ represents a hydrogen atom or an alkyl group, such as a methyl group, R⁷ represents an alkyl group containing from 1 to 10 carbon atoms, such as a methyl, ethyl, propyl or butyl group, R⁸ represents an alkylene group containing from 1 to 10 carbon atoms, b is an integer between 0 and 4, and c is 0 or 1 such that if c is 0, -(R⁸)_{c}- represents a bond.
R¹ represents an aryl group or an alkyl group containing from 1 to 10 carbon atoms, in which the alkyl group is preferably represented by a methyl, ethyl, propyl, butyl, pentyl, isopropyl, isobutyl, cyclopentyl or cyclohexyl group, and in which the methyl group is most preferable.

The copolymer comprising a carbosiloxane dendrimer structure may be the product of polymerization:
(A) of 0 to 99.9 parts by weight of a vinyl monomer; and
(B) from 100 to 0.1 parts by weight of a carbosiloxane dendrimer containing a group of general formula: 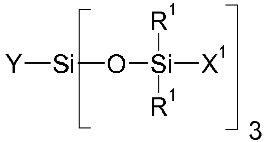 where Y, X¹ and R¹ are described as above.

The vinyl monomer may be chosen from the following monomers: methyl methacrylate, ethyl methacrylate, n-butyl methacrylate, methacrylic acid, cyclohexyl methacrylate, 2-ethylhexyl methacrylate, vinylpyrrolidone, itaconic acid, crotonic acid, fumaric acid, maleic acid or other vinyl monomers containing at least one carboxylic acid group.

The carbosiloxane dendrimer may be manufactured according to the manufacturing process described in Japanese Patent Application Hei 9-171 154.

The copolymer may be obtained by copolymerization of the components (A) and (B), or by polymerization of the component (B) on its own. The polymerization may be carried out in a solution over 3 to 20 hours in the presence of a radical initiator at a temperature ranging from 50°C to 150°C. A solvent suitable for this purpose is hexane, toluene or ethyl acetate.

The copolymer is preferably supplied in a linear or cyclic silicone oil (hexamethylcyclotrisiloxane, octamethylcyclotetrasiloxane, decamethylcyclopentasiloxane, dodecamethylcyclohexasiloxane) or in an organic oil (liquid paraffin, isoparaffin, isododecane).

According to one embodiment, the copolymer is one of the polymers described in the examples from Patent Application EP 0 963 751 or in the examples from Patent Application WO 03/045337.

A copolymer that can be used in the context of the present invention is available commercially under the references DOW CORNING FA 4002 ID or DOW CORNING FA 4001 CM which correspond respectively to ingredients of INCI name:
- ISODODECANE & ACRYLATES/POLYTRIMETHYLSILOXYMETHACRYLATE COPOLYMER and
- CYCLOPENTACYLOXANE & ACRYLATES/POLYTRIMETHYLSILOXYMETHACRYLATE COPOLYMER.

The copolymer is advantageously present in a content ranging from 3% to 85% by weight, relative to the total weight of the composition, preferably ranging from 5% to 25% by weight, preferably ranging from 5% to 10% by weight when it is a gloss or from 10 to 15% by weight when it is an eyeshadow.

### Polyester wax

The composition according to the invention contains at least one polyester wax.

The term "wax" is understood to mean, within the meaning of the present invention, a lipophilic compound with a reversible solid/liquid change of state, having a melting point greater than or equal to 25°C, which may range up to 100°C, and having in the solid state an anisotropic crystal organization.

Advantageously, the polyester wax has a melting point between 35°C and 95°C, especially between 40°C and 90°C.

Within the meaning of the invention, the melting point corresponds to the temperature of the most endothermic peak observed in thermal analysis (DSC) as described in the standard ISO 11357-3; 1999.

The melting point of the wax may be measured using differential scanning calorimetry (DSC), for example the calorimeter sold under the name DSC822 (equipped with STAR SW 8.10 measurement processing software) by Mettler. The measurement protocol is the following.

A sample of 10 to 15 mg of wax placed in a crucible is subjected to a first rise in temperature going from 0°C to 100°C, at a heating rate of 5°C/minute, a hold is made for 5 min at 100°C, then it is cooled from 100°C to 0°C at a cooling rate of 5°C/minute, then a hold is made at 0°C for 5 min, and it is finally subjected to a second rise in temperature going from 0°C to 100°C at a heating rate of 5°C/minute. During the rise in temperature, the difference is measured between the heat flow (heat absorbed per unit of time) of the wax sample and of the reference (empty crucible), which are subjected to the same temperature programme. The degree of crystallinity of the polyester wax at 25°C is equal to the ratio of the total amount of crystals present at a temperature of 25°C based on 200 J/g.

The degree of crystallinity of the polyester wax used in the context of the invention is preferably greater than 45%, more preferably greater than 60%. It is preferably less than 95%, more preferably less than 90%, or even less than 75%, for instance between 60 and 75%.

The polyester wax is, in one embodiment, a polyester of at least one polyol and of at least two, or even of at least three, identical or different, saturated or unsaturated, linear or branched, aliphatic monocarboxylic acid having, independently of one another, from 8 to 30 carbon atoms. Said acid preferably comprises from 12 to 30 carbon atoms, more preferably from 16 to 20 carbon atoms, and it may be optionally hydroxylated.
The aliphatic monocarboxylic acid is chosen, for example, from lauric, myristic, palmitic, oleic, stearic, isostearic, 12-hydroxystearic and behenic acids and mixtures thereof.
The polyol preferably comprises at least 2 hydroxyl groups, more preferably at least 3. The polyol may be chosen from glycerol or a glycerol condensate. The polyol may also be chosen from monosaccharides or polysaccharides comprising 1 to 10 oses, preferably 1 to 4, more preferably 1 or 2 oses. The polyol may be chosen from erythritol, xylitol, sorbitol, glucose, and sucrose.

According to one variant of the invention, the polyester wax is a sucrose ester of stearic acid and of acetic acid, preferably a sucrose ester such as that denoted by the INCI name Sucrose tetrastearate triacetate, sold in particular under the reference SISTERNA A 10E-C® marketed by SISTERNA.

In another embodiment, the polyester wax may especially be an ester obtained from: (i) a saturated or unsaturated, linear or branched, aliphatic monocarboxylic acid having from 8 to 30, and preferably from 12 to 22 carbon atoms, (ii) a linear or branched diacid having from 12 to 36, and preferably from 12 to 20 carbon atoms, and (iii) glycerol.
One glyceryl ester of this type, preferred for use in the present invention, is the diester of eicosadioic acid and of glycerol esterified by behenic acid. This compound is an ester of formula: RO-CH₂-CH(OR)-CH₂-O-CO-(CH₂)₁₈-CO-O-CH₂-CH(OR)-CH₂-OR, where the R groups independently denote a -CO-(CH₂)₂₀-CH₃ group or H, on condition that at least one of the R groups is other than H, or a mixture of such esters. It is, in particular, available from NISSHIN OILLIO under the trade name NOMCORT^{®} HK-G.
One glyceryl ester of this type, preferred for use in the present invention, is the diester of eicosadioic acid and of glycerol esterified by behenic acid and isostearic acid. This compound is an ester of formula: RO-CH₂-CH(OR)-CH₂-O-CO-(CH₂)₁₈-CO-O-CH₂-CH(OR)-CH₂-OR, where the R groups independently denote a -CO-(CH₂)₂₀-CH₃ group, -CO-C₁₇H₃₅ group or H, on condition that at least one of the R groups is other than H, or a mixture of such esters. It is, in particular, available from NISSHIN OILLIO under the trade name NOMCORT^{®} SG.

In yet another embodiment, the polyester wax is a derivative of beeswax selected from a silicone derivative, for example the reference Siliconyl Beeswax^{®}, manufactured by Koster Keunen. This polyester is obtained by esterification of beeswax and bears the INCI name: Bis-PEG-12 Dimethicone Beeswax.

In another embodiment, as a polyester wax, it is also possible to use a polyester obtained by reaction i) of a diacid dimer of isostearic acid with ii) an aliphatic alcohol. Such a wax is especially sold under the names KESTER WAX K 82 P^{®} and KESTER WAX K 80 P^{®} by KOSTER KEUNEN.
Mention will be made, for example, of alkyl (hydroxystearoyloxy)stearates and, in particular, a (C₂₀-C₄₀)alkyl 12-(12-hydroxystearoyloxy)stearate, of formula (I) : 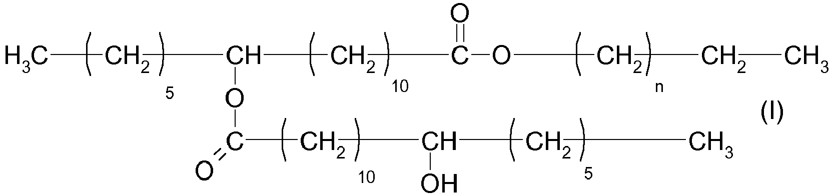 in which n is an integer ranging from 16 to 40.

The polyester wax may represent from 0.1 to 15% by weight, preferably from 0.6 to 15% by weight, more preferably from 1 to 10% by weight, more preferably still from 1.5 to 8% by weight, and even more preferably from 2 to 8% by weight relative to the total weight of the composition. The composition advantageously comprises a total amount of wax(es) that is less than or equal to 10%.

The polyester wax is preferably chosen from the following products (a trade name is indicated between parentheses):
- beeswax derivatives selected from the waxes of INCI name Synthetic Beeswax (Kester wax K82P®) and Bis-PEG-12 Dimethicone Beeswax (Siliconyl beeswax®);
- the product of INCI name Sucrose tetrastearate triacetate (Sisterna A10 CE®);
- the products of INCI names GLYCERYL TRIBEHENATE/ISOSTEARATE/EICOSANDIOATE (Nomcort SG®) and GLYCERYL TRIBEHENATE/EICOSANDIOATE (Nomcort HKG®);
- and mixtures thereof.

The following Table illustrates the technical features of suitable waxes that may be used in accordance with this invention.

| Trade name (INCI name) | Melting temperature (°C) | Cristallinity (%) |
|---|---|---|
| KESTER WAX K82P(SYNTHETIC BEESWAX) | 78-84 | 65.8 |
| SILICONYL BEESWAX | 62-72 | 68.5 |
| NOMCORT HK-G (GLYCERYL TRIBEHENATE/EICOSAND IOATE) | 65 | 71.8 |
| NOMCORT SG (GLYCERYL TRIBEHENATE/ISOSTEAR ATE/EICOSANDIOATE) | 58 | 65 |
| SISTERNA A10E-C (SUCROSE TETRASTEARATE TRIACETATE) | 44-50 | 49 |

The weight ratio of the acrylate/polytrimethylsiloxy-methacrylate copolymer to the polyester wax is advantageously between 10/1 and 1/1, preferably between 5/1 and 1/1.

### Volatile oil

The composition advantageously comprises at least one volatile oil.

Within the meaning of the present invention, the term "oil" is understood to mean a compound that is liquid at ambient temperature (25°C), and which, when it is introduced in an amount of at least 1% by weight into water at 25°C, is not at all soluble in the water, or is soluble up to less than 10% by weight, relative to the weight of oil introduced into the water.

The cosmetic oils that are volatile at ambient temperature especially have a vapour pressure, measured at ambient temperature and atmospheric pressure, ranging from 10⁻³ mmHg to 300 mmHg (0.266 Pa to 40 000 Pa), preferably from 0.02 mmHg to 300 mmHg (2.66 Pa to 40 000 Pa) and better still ranging from 0.1 mmHg to 90 mmHg (13 Pa to 12 000 Pa).

The volatile oil may be chosen from oils that do not have a flash point, oils that have a flash point ranging from 40°C to 100°C, and mixtures thereof, with a view to facilitating the implementation thereof. Moreover, they advantageously have a boiling point at atmospheric pressure below 220°C and better still below 210°C, especially ranging from 110 to 210°C. Preferably, these volatile oils are not monoalcohols having at least 7 carbon atoms.

As a volatile oil that can be used in the invention, mention may be made of linear or cyclic silicones oils that have a viscosity at ambient temperature of less than 8 cSt and that especially have from 2 to 7 silicon atoms, these silicones optionally comprising alkyl or alkoxy groups having from 1 to 10 carbon atoms. As a volatile silicone oil that can be used in the invention, mention may especially be made of octamethylcyclotetrasiloxane, decamethylcyclopentasiloxane, dodecamethylcyclohexasiloxane, heptamethylhexyltrisiloxane, heptamethyloctyl-trisiloxane, hexamethyldisiloxane, octamethyltrisiloxane, decamethyltetrasiloxane, dodecamethylpentasiloxane and mixtures thereof.

As another volatile oil that can be used in the invention, mention may be made of volatile hydrocarbon-based oils having from 8 to 16 carbon atoms and mixtures thereof, and especially C₈-C₁₆ branched alkanes such as C₈-C₁₆ isoalkanes (also called isoparaffins), isododecane, isodecane, isohexadecane and, for example, the oils sold under the trade names Isopar^{®} or Permetyl^{®}, and mixtures thereof.

Preferably, use is made of isododecane (Permetyls 99 A), C₈-C₁₆ isoparaffins such as Isopar L, E, G or H, mixtures thereof, optionally combined with decamethyltetrasiloxane or cyclopentasiloxane.

These volatile oils especially represent from 0.1 to 80% of the total weight of the composition, and better still from 10 to 75%, preferably between 20 and 50% of the total weight of the composition, for example from 30 to 40%, preferably around 35% of the total weight of the composition.

### Non-volatile oil

The composition according to the invention may comprise from 1 to 60% by weight of at least one non-volatile oil, especially from 5 to 20%, relative to the total weight of the composition.

The oils that can be used in the composition according to the invention may be chosen from: hydrocarbon-based oils; synthetic (poly)esters and (poly)ethers and in particular the (poly) esters of C₆-C₂₀ acids and of C₆-C₂₀ alcohols that are advantageously branched, such as isononyl isononanoate; plant oils; branched and/or unsaturated fatty acids; branched and/or unsaturated fatty alcohols such as octyldodecanol; silicone oils such as linear, optionally phenylated, or cyclic polydimethylsiloxanes; fluorosilicone oils; fluorinated oils; and mixtures thereof.

It is preferred to incorporate into the composition at least one oil having a molar mass ranging in particular from 650 to 10 000 g/mol, and preferably from 750 to 7500 g/mol. Such an oil may be qualified as a shiny oil.
The oil having a molar mass ranging from 650 to 10 000 g/mol may be chosen from:
- polybutylenes such as INDOPOL H-100^{®} (molar mass or MM = 965 g/mol), INDOPOL H-300^{®} (MM = 1340 g/mol), INDOPOL H-1500^{®} (MM = 2160 g/mol) sold or manufactured by AMOCO, hydrogenated polyisobutylenes such as PANALANE H-300 E^{®} sold or manufactured by AMOCO (MM = 1340 g/mol), VISEAL 20000^{®} sold or manufactured by SYNTEAL (MM = 6000 g/mol), REWOPAL PIB 1000^{®} sold or manufactured by WITCO (MM = 1000 g/mol);
- polydecenes and hydrogenated polydecenes such as: PURESYN 10^{®} (MM = 723 g/mol), PURESYN 150^{®} (MM = 9200 g/mol) sold or manufactured by MOBIL CHEMICALS;
- vinylpyrrolidone copolymers such as: the vinylpyrrolidone/1-hexadecene copolymer, ANTARON V-216^{®} sold or manufactured by ISP (MM = 7300 g/mol);

- silicone oils such as phenylated silicones for instance BELSIL PDM 1000^{®} from WACKER (MM = 9000 g/mol), phenylated silicone oils such as those identified by the INCI name "phenyl trimethicone", an example of which is constituted by the silicone available under the trade name MIRASIL^{®} PTM from RHODIA, those identified by the INCI name "phenylpropyldimethylsiloxysilicate", an example of which is constituted by the silicone available under the trade name SILSHINE^{®} 151 from GENERAL ELECTRIC and those identified by the INCI name "trimethyl-pentaphenyltrisiloxane", an example of which is constituted by the silicone available under the trade name DC PH^{®} 1555 HRI from DOW CORNING;
- and mixtures thereof.

As shiny oils, mention may also be made of the fluorosilicones identified by the INCI name perfluorononyl dimethicone, an example of which is constituted by the silicone available under the trade name PECOSIL^{®} FS (FSU, FSL...) from PHOENIX and another example is constituted by the silicone available under the trade name Biosil Basics^{®} (Fluorosil LF, 14...) from BIOSIL TECHNOLOGIES.

Other examples of shiny oils are natural oils and in particular castor seed oil; monoesters and polyesters of fatty acids and/or of fatty alcohols, the fatty chain of which contains from 6 to 20 carbon atoms, and in particular: monoesters and polyesters of hydroxy acids and of fatty alcohols such as diisostearyl malate, esters of benzoic acid and of fatty alcohols such as C₁₂-C₁₅ alkyl benzoate, the polyesters of polyols and especially of (di)pentaerythrityl, such as pentaerythrityl tetra-isostearate, dipentaerythrityl pentaisononanoate and C₅-C₉ esters of dipentaerythrityl, or of polyglycerol, such as the one known under the INCI name "bis-diglyceryl polyacyladipate-1" and sold by SASOL under the trade name SOFTISAN^{®} 645, or of trimethylolpropane, such as trimethylolpropane triethylhexanoate, which is especially sold by KOKYU ALCOHOL KOGYO under the trade name KAK^{®} TTO, or of propylene glycol, such as propylene glycol dibenzoate, which is especially sold by INOLEX under the trade name LEXFEEL SHINE^{®}, and isocetyl stearoyl stearate; and the polyesters of hydrogenated castor oil such as the esters sold by KOKYU ALCOHOL KOGYO under the trade names RISOCAST^{®} DA-H and RISOCAST^{®} DA-L.

According to one particular embodiment, the composition comprises polybutylene or diisostearyl malate as a non-volatile oil.

The composition according to the invention advantageously contains from 5 to 50% by weight, in particular from 10 to 40% by weight, preferably from 15 to 35% by weight of at least one oil having a molar mass ranging, in particular, from 650 to 10 000 g/mol, relative to the total weight of the composition.

### Gelling agents

The composition according to the invention advantageously contains a gelling agent.
Among the gelling agents that can be used are block copolymers that contain styrene and at least one olefin which may be chosen from: ethylene, propylene, butylene, butadiene and isoprene, without this list being limiting.

It may thus be a copolymer sold by SHELL under the trade name KRATON^{®}, which may especially be an ethylene/propylene/styrene or butylene/ethylene/styrene copolymer or mixtures thereof.

These copolymers are generally sold as a dispersion in at least one oil chosen, for example, from: a volatile or non-volatile hydrocarbon, such as a mineral oil, hydrogenated polyisobutene, hydrogenated polydecene, the isohexadecane or isododecane; or a fatty acid ester such as isopropyl palmitate or isononyl isononanoate; or a benzoic acid ester. According to the invention, it is preferred that the styrene/olefin copolymer be carried in hydrogenated polyisobutene.

Another example of a gelling agent that can be used according to the invention is constituted by the DEKAGEL^{®} compounds sold by JAN DEKKER, and in particular DEKAGEL^{®} HV2004.

Preferred gelling agents for use in the present invention are those sold by PENRECO under the trade names VERSAGEL^{®} M, ME, ML, MP, MC, MD and MN. Those from the ME series are particularly preferred.
These copolymers advantageously have a weight-average molecular weight ranging from 50 000 to 260 000. Moreover, the anhydrous gel which contains them has a viscosity, at 25°C, measured on a Brookfield viscometer using the T-C spindle at 5 rpm after three days of conditioning at rest at ambient temperature, which is greater than 180 000 cPs, preferably greater than 200 000 cPs, more preferably greater than 220 000 cPs, better still greater than 240 000 cPs, such as a viscosity of around 250 000 cPs. It is particularly preferred to use the copolymer sold by PENRECO under the trade name VERSAGEL^{®} ME 2000.

Examples of gelling agents are especially silicone polymers and more particularly organopolysiloxane elastomers. Examples of such elastomers are especially sold by SHIN ETSU under the trade names KSG-6, KSG-16, KSG-31, KSG-32, KSG-41, KSG-42, KSG-43, KSG-44 KSG-21 and KSG-210, by DOW CORNING under the trade names DC 9040 and DC 9041 and by GRANT INDUSTRIES under the trade name Gransil^{®}. Another gelling agent is constituted by a silicone polymer, obtained by self-polymerization of an organopolysiloxane functionalized by epoxy and hydrosilyl groups, in the presence of a catalyst, which is commercially available from GENERAL ELECTRIC under the trade name VELVESIL^{®} 125. Another gelling agent is constituted by a cyclic vinyldimethicone/dimethicone copolymer such as that sold by JEEN under the trade name JEESILC^{®} PS (including PS-VH, PS-VHLV, PS-CM, PS-CMLV and PS-DM). Another type of gelling agent is constituted by polyamides such as those identified by the INCI name polyamide-3 and in particular the polymers SYLVACLEAR^{®} AF 1900V and PA 1200V available from ARIZONA CHEMICAL and also those identified by the INCI name "Ethylenediamine/Hydrogenated Dimer Dilinoleate Copolymer Bis-Di-C14-18 Alkyl Amide" and available, for example, under the trade name SYLVACLEAR^{®} A200V or SYLVACLEAR^{®} A2614V from ARIZONA CHEMICAL.
Another gelling agent is a sucrose ester of isobutyric acid and of acetic acid, such as the product sold under the reference Sustane SAIB MCT^{®} (INCI name sucrose acetate isobutyrate).

As another gelling agent, mention may be made of optionally modified clays, such as hectorites modified by a C₁₀ to C₂₂ fatty acid ammonium chloride, such as the hectorite modified by distearyl dimethyl ammonium chloride such as, for example, that sold under the name Bentone 38V® by ELEMENTIS.

Mention may also be made of fumed silica optionally subjected to a hydrophobic surface treatment, the particle size of which is less than 1 µm. Specifically, it is possible to chemically modify the surface of the silica, by chemical reaction generating a reduced number of silanol groups present at the surface of the silica. It is especially possible to substitute silanol groups with hydrophobic groups: a hydrophobic silica is then obtained. The hydrophobic groups may be:
trimethylsiloxyl groups, which are obtained especially by treating fumed silica in the presence of hexamethyldisilazane. Silicas thus treated are known as "Silica silylate" according to the CTFA. They are sold, for example, under the references Aerosil R812^{®} by DEGUSSA, and Cab-O-Sil TS-530^{®} by CABOT; dimethyl-silyloxyl or polydimethylsiloxane groups, which are obtained especially by treating fumed silica in the presence of polydimethylsiloxane or dimethyldichlorosilane. Silicas thus treated are known as 'Silica dimethyl silylate'. They are sold, for example, under the references Aerosil R972^{®} and Aerosil R974^{®} by DEGUSSA, and Cab-O-Sil TS-610^{®} and Cab-O-Sil TS-720^{®} by CABOT. The hydrophobic fumed silica in particular has a particle size that may be of nanometre to micrometre scale, for example ranging from about 5 to 200 nm.

The composition may also comprise at least one film-forming polymer capable of introducing hold and/or transfer-free properties and/or sheen to the makeup conferred by the composition. It may especially be a silicone polymer optionally modified by urethane or fluorine or acrylate, such as the (meth)acrylate silicones sold by SHIN-ETSU under the trade names KP-545^{®}, KP-561^{®} and KP-562^{®}. Other examples of film-forming polymers are silicone resins and in particular MQ resins, such as trimethylsiloxysilicates, and MT resins, such as silsesquioxane derivatives and in particular polymethyl-silsesquioxanes, sold in particular by SHIN-ETSU, and also the polypropylsilsesquioxanes sold by DOW CORNING under the trade name DC 670^{®} or the phenylpropylpolysilsesquioxane sold by WACKER under the trade name BELSIL SPR45VP^{®}. Another example is constituted by fluorosilicone polymers identified by the INCI name "Trifluoropropyldimethylsiloxy Triethylsiloxy-silicate", such as that sold by General Electric under the trade name XS66-B8226^{®}. Use may also be made, as film-forming polymers, of bioadhesive polymers obtained, for example, by polycondensation of dimethiconol and of MQ silicate resin in a solvent, such as heptane, which are sold in particular by DOW CORNING under the trade names DC 7-4405 low tack^{®} and DC 7-4505 high tack^{®}. Other examples of film-forming polymers are poly(cyclic olefins), such as polycyclopentadiene, sold in particular by KOBO under the trade name KOBOGUARD 5400, or else polydicyclopentadiene. Yet other examples of film-forming polymers are constituted by copolymers of vinylpyrrolidone (VP) and/or of linear olefins, such as VP/hexadecene and VP/eicosene copolymers, including ANTARON V216^{®} and ANTARON V220^{®} from ISP, or else ethylene/vinyl acetate copolymers, such as AC 400 from BAERLOCHER. Mention may also be made of polyethers, such as poly(vinyl stearyl ether) sold in particular by PHOENIX under the trade name GIOVARE2^{®} 1800. Other film-forming polymers capable of being used in this invention are polyacrylates, such as poly(ethyl acrylate), sold in particular by CREATIONS COULEURS under the trade name CREASIL 7 ID^{®}.

It is preferred that the composition according to the invention contains less than 5% by weight of water, preferably less than 3% by weight.

The composition used according to the invention may additionally contain at least one filler. The term "filler" is understood to mean any inorganic or organic particle of any shape (especially spherical or lamellar) that is insoluble in the composition. Examples of fillers are talc, mica, silica, kaolin, boron nitride, starch, starch modified by octenylsuccinic anhydride, polyamides, silicone resins, powders of silicone elastomers and powders of acrylic polymers, in particular of poly(methyl methacrylate). The fillers may especially be composed of several layers of different chemical nature and/or of different physical form and may in particular be in the form of lamellae coated with spherical fillers. They may be modified using various surface treatments. An example of a surface-treated filler is composed of silica modified by an ethylene/methacrylate copolymer, sold in particular by KOBO under the trade names DSPCS^{®} 20N-I2, 3H-I2 and I2.

The composition may also contain at least one colorant chosen from water-soluble or liposoluble dyes, fillers having the effect of colouring and/or opacifying the composition and/or of colouring the lips, such as pigments, pearlescent agents, lakes (water-soluble dyes adsorbed on an inert inorganic support), and mixtures thereof. These colorants may optionally be surface-treated with a hydrophobic agent, such as silanes, silicones, fatty acid soaps, C₉-C₁₅ fluoroalkyl phosphates, acrylate/dimethicone copolymers, mixed C₉-C₁₅ fluoroalkyl phosphate/silicone copolymers, lecithins, carnauba wax, polyethylene, chitosan and optionally acylated amino acids, such as lauroyl lysine, disodium stearoyl glutamate and aluminium acyl glutamate. The pigments may be inorganic or organic and natural or synthetic. Examples of pigments are in particular iron, titanium or zinc oxides, and also composite pigments and goniochromatic, pearlescent, interference, photochromic or thermochromic pigments, without this list being limiting. Examples of pigments that can be used in the composition according to the invention are hemispherical composite pigments manufactured from crosslinked poly(methacrylic acid) methyl ester and organic dyes. Such composite pigments are sold in particular by DAITO KASEI. The pearlescent agents may be chosen from those conventionally present in makeup products, such as mica/titanium dioxide products. As a variant, they may be pearlescent agents based on mica/silica/titanium dioxide, based on synthetic fluorphlogopite/titanium dioxide (SUNSHINE^{®} from MAPRECOS), on calcium sodium borosilicate/titanium dioxide (REFLECKS^{®} by ENGELHARD) or on calcium aluminium borosilicate/silica/titanium dioxide (RONASTAR^{®} from MERCK).

Advantageously, when it contains one or more pigments, the composition according to the invention additionally contains at least one dispersant such as butylene glycol cocoate or diisostearyl malate.

The composition according to the invention may also contain one or more sweetening agents, such as sorbitol, sucrose, xylitol, acesulfame K and sodium saccharinate; antioxidants, such as ascorbic acid and/or its alkyl or phosphoryl esters, or else tocopherol and its esters; sequestering agents, such as EDTA salts; pH adjusters; preservatives; and fragrances.

It may in addition contain at least one UV screening agent chosen from organic and inorganic screening agents and mixtures thereof. Mention may especially be made, as organic screening agents, of dibenzoylmethane derivatives (including butyl methoxydibenzoylmethane), cinnamic acid derivatives (including ethylhexyl methoxycinnamate), salicylates, para-aminobenzoic acids, β,β'-diphenylacrylates, benzophenones, benzylidenecamphor derivatives, phenylbenzimidazoles, triazines, phenyl-benzotriazoles and anthranilic derivatives. Mention may especially be made, as inorganic screening agents, of screening agents based on inorganic oxides in the form of pigments or nanopigments which may or may not be coated and in particular based on titanium dioxide or on zinc oxide.

Examples of such adjuvants are mentioned in particular in the CTFA dictionary (International Cosmetic Ingredient Dictionary and Handbook published by The Cosmetic, Toiletry and Fragrance Association, 12th Edition, 2008).

Preferably, the composition employed according to the invention is used as a product for caring for or making up the skin, including the lips, for example as lip gloss, as lipstick, concealer or eyeshadow.

Another subject of the present invention is therefore a cosmetic method for caring for or making up the lips, comprising the topical application of the composition as described previously to the skin.

The invention will now be illustrated by the following non-limiting examples.

**Example 1: Eyeshadow**

| Trade name | INCI name | (%) |
|---|---|---|
| DOW CORNING FA 4001 CM SILICONE ACRYLATE | CYCLOPENTASILOXANE & ACRYLATES/POLYTRIMETHYL-SILOXYMETHACRYLATE COPOLYMER | 60.0 |
| DUB ININ | ISONONYL ISONONANOATE | 5.0 |
| KF-995 | CYCLOMETHICONE | 10.0 |
| CRYSTALLINE POLYESTER* | - | 5.0 |
| TIMIRON STARLUSTER MP 115 (REF 17200) | MICA & TITANIUM DIOXIDE | 9.0 |
| TIMIRON SPLENDID GOLD | CI 77891 (TITANIUM DIOXIDE) & MICA & SILICA | 6.5 |
| COLORONA BRONZE FINE | MICA & IRON OXIDE | 4.5 |
| | | 100.0 |

The crystalline polyester is one of the following polyesters:

| Trade name (INCI name) | Melting point (°C) | Degree of crystallinity(%) |
|---|---|---|
| KESTER WAX K82P (SYNTHETIC BEESWAX) | 78-84 | 65.8 |
| SILICONYL BEESWAX | 62-72 | 68.5 |
| NOMCORT SG (GLYCERYL TRIBEHENATE/ISOSTEARATE/ EICOSANDIOATE) | 58 | 65 |

**Example 2: Gloss**

| Trade name/ chemical nature | INCI name | (%) |
|---|---|---|
| DOW CORNING FA 4002 ID | ISODODECANE & ACRYLATES/POLYTRIMETHYL-SILOXYMETHACRYLATE COPOLYMER | 40.0 |
| CRODADERM S | SUCROSE POLYSOYATE | 1.1 |
| DL-ALPHA-TOCOPHERYL ACETATE | TOCOPHERYL ACETATE | 0.5 |
| pigments | - | 1.2 |
| OXYNEX K | PEG-8 & TOCOPHEROL & ASCORBYL PALMITATE & ASCORBIC ACID & CITRIC ACID | 0.15 |
| KESTER WAX K82P | SYNTHETIC BEESWAX | 2.5 |
| BENTONE GEL GTCC V | CAPRYLIC/CAPRIC TRIGLYCERIDE & STEARALKONIUM HECTORITE & PROPYLENE CARBONATE | 10.0 |
| AEROSIL R 972 | SILICA DIMETHYL SILYLATE | 4.5 |
| MIGLYOL 812 | CAPRYLIC/CAPRIC TRIGLYCERIDE | 40.05 |
| | | **100.0** |

The rheological profile of the composition was measured under the conditions described previously.
The average elastic modulus G' in the linear area of the curve G'(σ) of Example 2 was equal to 1072 ± 75 Pa, and the average viscous modulus G'' in the linear area of the curve G''(σ) measured on the compositions of the invention was equal to 306 ± 139 Pa.

The same composition was produced, replacing, weight for weight, the crystalline polyester of trademark Kester wax K82P® (melting point equal to 79-84°C and degree of crystallinity equal to 65.8%) with the Cera Alba beeswax of trademark Cerabeil blanche^{®} (melting point equal to 61-65°C and degree of crystallinity equal to 99.5%).

The composition of Example 2 and the comparative example containing the beeswax were applied to half-lips. After application, drying for 5 minutes and transfer to the back of the hand, the formula containing the beeswax transferred more, and the film remaining on the lips was less uniform; moreover, the parts transferred left "holes".

**Example 3: Gloss**

| Trade name/ chemical nature | INCI name | (%) |
|---|---|---|
| DOW CORNING FA 4002 ID | ISODODECANE & ACRYLATES/POLYTRIMETHYL-SILOXYMETHACRYLATE COPOLYMER | 30.0 |
| POLYBUTENE M 100 | POLYBUTENE | 10.0 |
| SALACOS 222 | DIISOSTEARYL MALATE | 14.9 |
| DL-ALPHA-TOCOPHERYL ACETATE | TOCOPHERYL ACETATE | 0.5 |
| pigments | - | 5.2 |
| OXYNEX K | PEG-8 & TOCOPHEROL & ASCORBYL PALMITATE & ASCORBIC ACID & CITRIC ACID | 0.2 |
| NOMCORT SG | GLYCERYL TRIBEHENATE/ ISOSTEARATE/EICOSANDIOATE | 4.0 |
| DEKAGEL HV 2004 | Hydrogenated polyisobutene & ethylene/propylene/styrene copolymer & butylene/ethylene/ styrene copolymer | 10.0 |
| MIGLYOL 812 | CAPRYLIC/CAPRIC TRIGLYCERIDE | 25.0 |
| Preservatives | | 0.2 |
| | | 100.0 |

**Example 4: Gloss**

| Trade name/ chemical nature | INCI name | (%) |
|---|---|---|
| DOW CORNING FA 4001 CM | CYCLOPENTACYLOXANE& ACRYLATES/POLYTRIMETHYLSILOXY METHACRYLATE COPOLYMER | 30.0 |
| BENTONE GEL GTCC V | CAPRYLIC/CAPRIC TRIGLYCERIDE & STEARALKONIUM HECTORITE & PROPYLENE CARBONATE | 5.0 |
| SUSTANE SAIB MCT | SUCROSE ACETATE ISOBUTYRATE | 20.0 |
| SALACOS 222 | DIISOSTEARYL MALATE | 31.7 |
| DL-ALPHA-TOCOPHERYL ACETATE | TOCOPHERYL ACETATE | 0.5 |
| pigments | - | 2.1 |
| OXYNEX K | PEG-8 & TOCOPHEROL & ASCORBYL PALMITATE & ASCORBIC ACID & CITRIC ACID | 0.2 |
| NOMCORT SG | GLYCERYL TRIBEHENATE/ ISOSTEARATE/EICOSANDIOATE | 5.5 |
| MIGLYOL 812 | CAPRYLIC/CAPRIC TRIGLYCERIDE | 5.0 |
| Preservatives | | 0.3 |

The rheological profile of the composition was measured under the conditions described previously.
The average elastic modulus G' in the linear area of the curve G'(σ) of Example 2 was equal to 30 711 ± 2850 Pa, and the average viscous modulus G'' in the linear area of the curve G''(σ) measured on the compositions of the invention was equal to 4894 ± 539 Pa.

**Example 5: Gloss**

| Trade name/ chemical nature | INCI name | (%) |
|---|---|---|
| DOW CORNING FA 4002 ID | ISODODECANE & ACRYLATES/POLYTRIMETHYLSILOXYM ETHACRYLATE COPOLYMER | 30.0 |
| POLYBUTENE M 100 | POLYBUTENE | 2.0 |
| SUSTANE SAIB MCT | SUCROSE ACETATE ISOBUTYRATE | 10.0 |
| SALACOS 222 | DIISOSTEARYL MALATE | 34.4 |
| DL-ALPHA-TOCOPHERYL ACETATE | TOCOPHERYL ACETATE | 0.5 |
| pigments | - | 2.4 |
| OXYNEX K | PEG-8 & TOCOPHEROL & ASCORBYL PALMITATE & ASCORBIC ACID & CITRIC ACID | 0.2 |
| NOMCORT SG | GLYCERYL TRIBEHENATE/ ISOSTEARATE/EICOSANDIOATE | 5.5 |
| MIGLYOL 812 | CAPRYLIC/CAPRIC TRIGLYCERIDE | 15.0 |
| Preservatives | | 0.2 |

**Example 6: Gloss**

| Trade name/ chemical nature | INCI name | (%) |
|---|---|---|
| DOW CORNING FA 4002 ID | ISODODECANE & ACRYLATES/POLYTRIMETHYLSILOXY METHACRYLATE COPOLYMER | 30.0 |
| POLYBUTENE M 100 | POLYBUTENE | 10.0 |
| SALACOS 222 | DIISOSTEARYL MALATE | 14.9 |
| DL-ALPHA-TOCOPHERYL ACETATE | TOCOPHERYL ACETATE | 0.5 |
| pigments | - | 5.2 |
| OXYNEX K | PEG-8 & TOCOPHEROL & ASCORBYL PALMITATE & ASCORBIC ACID & CITRIC ACID | 0.15 |
| NOMCORT SG | GLYCERYL TRIBEHENATE/ ISOSTEARATE/EICOSANDIOATE | 4.0 |
| DEKAGEL HV 2004 | Hydrogenated polyisobutene & ethylene/propylene/styrene copolymer & butylene/ethylene/ styrene copolymer | 10.0 |
| MIGLYOL 812 | CAPRYLIC/CAPRIC TRIGLYCERIDE | 25.0 |
| Preservatives | | 0.2 |

**Example 7: Long-lasting gloss**

| Trade name/ chemical nature | INCI name | (%) |
|---|---|---|
| DOW CORNING FA 4001 CM | CYCLOPENTACYLOXANE& ACRYLATES/POLYTRIMETHYLSILOXY METHACRYLATE COPOLYMER | 30.0 |
| BENTONE GEL GTCC V | CAPRYLIC/CAPRIC TRIGLYCERIDE & STEARALKONIUM HECTORITE & PROPYLENE CARBONATE | 5.0 |
| SUSTANE SAIB MCT | SUCROSE ACETATE ISOBUTYRATE | 20.0 |
| SALACOS 222 | DIISOSTEARYL MALATE | 24.1 |
| DL-ALPHA-TOCOPHERYL ACETATE | TOCOPHERYL ACETATE | 0.5 |
| Pigments | - | 3.9 |
| OXYNEX K | PEG-8 & TOCOPHEROL & ASCORBYL PALMITATE & ASCORBIC ACID & CITRIC ACID | 0.2 |
| NOMCORT HK-G | GLYCERYL BEHENATE/ EICOSANDIOATE | 1.0 |
| MIGLYOL 812 | CAPRYLIC/CAPRIC TRIGLYCERIDE | 15.0 |
| Preservatives | | 0.3 |

The same composition was produced, replacing, weight for weight, the crystalline polyester of trademark NOMCORT HK-G ® (melting point equal to 65°C and degree of crystallinity equal to 71.8%) with the Cera Alba beeswax of trademark Cerabeil blanche^{®} (melting point equal to 61-65°C and degree of crystallinity equal to 99.5%).

The composition of the comparative example containing the beeswax was not stable (tendency to phase separate).

## Claims

1. Cosmetic composition for making up or caring for the skin comprising, in a physiologically acceptable medium:
(a) 0.6 to 20% by weight relative to the total weight of said composition of at least one polyester wax having a melting point between 40 and 90°C, and the degree of crystallinity of which is less than 95%, preferably less than 90% or even less than 75%, and may be greater than 45%;
wherein the degree of crystallinity of the polyester wax at 25°C is equal to the ratio of the total amount of crystals present at a temperature of 25°C based on 200 J/g
said polyester wax being chosen from:
- a polyester of at least one polyol and of at least two, or even of at least three, identical or different, saturated or unsaturated, linear or branched, aliphatic monocarboxylic acids having, independently of one another, from 8 to 30 carbon atoms,
- the polyester wax obtained from (i) a saturated or unsaturated, linear or branched, aliphatic monocarboxylic acid having from 8 to 30 carbon atoms; (ii) a linear or branched diacid having from 12 to 36 carbon atoms; and (iii) glycerol,
- beeswax derivatives selected from the waxes having the INCI name Synthetic Beeswax, Bis-PEG-12 Dimethicone Beeswax, and mixtures thereof, and
- polyester obtained by reaction i) of a diacid dimer of isostearic acid with ii) an aliphatic alcohol,
and
(b) from 1 to 25% relative to the total weight of said composition of at least one acrylate/poly-trimethylsiloxymethacrylate copolymer.

2. Cosmetic composition according to Claim 1, **characterized in that** said polyester wax and said copolymer are in an amount such that said composition has, at 20°C in the ambient atmosphere:
- an average elastic modulus G' between 0.1 and 120 000 Pa; and
- an average viscous modulus G'' between 30 and 30 000 Pa.

3. Composition according to Claim 1, **characterized in that** the polyester wax is obtained from (i) said aliphatic monocarboxylic acid, chosen from lauric, myristic, palmitic, oleic, stearic, 12-hydroxystearic and behenic acids and mixtures thereof ; (ii) a linear or branched diacid having from 12 to 36 carbon atoms chosen from eicosadioic, ethyloctadecanedioic and dodecanedioic acids and mixtures thereof ; and (iii) glycerol.

4. Composition according to the preceding Claims, **characterized in that** the polyester wax is a diester of eicosadioic acid and of glycerol esterified by behenic acid, and optionally by isostearic acid.

5. Composition according to the preceding claims, **characterized in that** the polyester wax is a sucrose ester of stearic acid and of acetic acid.

6. Composition according to any one of Claims 1 to 5, **characterized in that** the polyester wax represents from 0.6 to 15% by weight, preferably from 1 to 10% by weight, more preferably from 2 to 8% by weight of the weight of the composition.

7. Composition according to any one of the preceding claims, **characterized in that** the weight ratio of the acrylate/polytrimethylsiloxymethacrylate copolymer to the polyester wax is between 5/1 and 1/1.

8. Composition according to any one of the preceding claims, **characterized in that** it comprises from 0.1 to 80% by weight relative to the weight of the composition of at least one volatile oil, such as isododecane or cyclopentadisiloxane.

9. Composition according to any one of the preceding claims, **characterized in that** it comprises at least one non-volatile oil having a molecular weight greater than 650 g/mol.

10. Composition according to any one of the preceding claims, **characterized in that** it comprises less than 5% by weight of water.

11. Composition according to one of the preceding claims, **characterized in that** its average elastic modulus G' is between 0.1 and 120 000 Pa, preferably between 100 and 50 000 Pa, more preferably between 700 and 35 000 Pa.

12. Composition according to one of the preceding claims, **characterized in that** its average viscous modulus G'' is between 30 and 30 000 Pa, preferably between 100 and 10 000 Pa, more preferably between 300 and 5000 Pa.

13. Composition according to any one of the preceding claims, **characterized in that** it is in the form of an eyeshadow, a lipgloss, a lipstick, an eyeliner or a face powder.

## Patentansprüche

1. Kosmetische Zusammensetzung für Make-up oder zur Hautpflege umfassend, in einem physiologisch annehmbaren Medium:
(a) 0,6 bis 20 Gew.-%, bezogen auf das Gesamtgewicht der Zusammensetzung, von mindestens einem Polyesterwachs mit einem Schmelzpunkt zwischen 40 und 90 °C, wobei dessen Kristallinitätsgrad kleiner als 95 %, bevorzugt kleiner als 90 % oder sogar kleiner als 75 % ist und größer als 45 % sein kann;
wobei der Kristallinitätsgrad des Polyesterwachses bei 25 °C gleich dem Anteil an der Gesamtmenge an bei einer Temperatur von 25 °C vorliegenden Kristallen basierend auf 200 J/g ist
wobei das Polyesterwachs ausgewählt ist aus:
- einem Polyester aus mindestens einem Polyol und aus mindestens zwei, oder sogar aus mindestens drei, identischen oder unterschiedlichen, gesättigten oder ungesättigten, linearen oder verzweigten, aliphatischen Monocarbonsäuren mit, unabhängig voneinander, 8 bis 30 Kohlenstoffatomen,
- dem Polyesterwachs erhalten aus (i) einer gesättigten oder ungesättigten, linearen oder verzweigten, aliphatischen Monocarbonsäure mit 8 bis 30 Kohlenstoffatomen; (ii) einem linearen oder verzweigten Diacid mit von 12 bis 36 Kohlenstoffatomen; und (iii) Glycerin,
- Bienenwachsderivaten ausgewählt aus den Wachsen mit den INCI Namen Synthetisches Bienenwachs, Bis-PEG-12 Dimethicon Bienenwachs und Gemischen davon, und
- Polyester erhalten durch Umsetzen i) eines Diaciddimers von Isostearinsäure mit ii) einem aliphatischen Alkohol,
und
(b) von 1 bis 25 %, bezogen auf das Gesamtgewicht der Zusammensetzung, von mindestens einem Acrylat/Polytrimethylsiloxymethacrylat-Copolymer.

2. Kosmetische Zusammensetzung nach Anspruch 1, **dadurch gekennzeichnet, dass** das Polyesterwachs und das Copolymer in einer solchen Menge vorliegen, dass die Zusammensetzung, bei 20 °C in Umgebungsatmosphäre:
- ein mittleres Elastizitätsmodul G' zwischen 0,1 und 120.000 Pa; und
- ein mittleres Viskositätsmodul G" zwischen 30 und 30.000 Pa aufweist.

3. Zusammensetzung nach Anspruch 1, **dadurch gekennzeichnet, dass** das Polyesterwachs erhalten wird aus (i) der aliphatischen Monocarbonsäure, ausgewählt aus Laurinsäure, Myristinsäure, Palmitinsäure, Ölsäure, Stearinsäure, 12-Hydroxy-stearinsäure und Behensäure und Gemischen davon; (ii) einem linearen oder verzweigten Diacid mit von 12 bis 36 Kohlenstoffatomen ausgewählt aus Eicosadicarbonsäure, Ethyloctadecandicarbonsäure und Dodecandicarbonsäure und Gemischen davon; und (iii) Glycerin.

4. Zusammensetzung nach einem der vorhergehenden Ansprüche, **dadurch gekennzeichnet, dass** das Polyesterwachs ein Diester aus Eicosadicarbonsäure und aus Glycerin, verestert mit Behensäure, und optional mit Isostearinsäure, ist.

5. Zusammensetzung nach einem der vorhergehenden Ansprüche, **dadurch gekennzeichnet, dass** das Polyesterwachs ein Saccharoseester von Stearinsäure und von Essigsäure ist.

6. Zusammensetzung nach einem der Ansprüche 1 bis 5, **dadurch gekennzeichnet, dass** das Polyesterwachs von 0,6 bis 15 Gew.-%, bevorzugt von 1 bis 10 Gew.-%, mehr bevorzugt von 2 bis 8 Gew.-% des Gewichts der Zusammensetzung darstellt.

7. Zusammensetzung nach einem der vorhergehenden Ansprüche, **dadurch gekennzeichnet, dass** das Gewichtsverhältnis des Acrylat/Polytrimethylsiloxymethacrylat-Copolymers zum Polyesterwachs zwischen 5/1 und 1/1 beträgt.

8. Zusammensetzung nach einem der vorhergehenden Ansprüche, **dadurch gekennzeichnet, dass** sie von 0,1 bis 80 Gew.-%, bezogen auf das Gewicht der Zusammensetzung, von mindestens einem flüchtigen Öl, wie etwa Isododecan oder Cyclopentadisiloxan, umfasst.

9. Zusammensetzung nach einem der vorhergehenden Ansprüche, **dadurch gekennzeichnet, dass** sie mindestens ein nicht-flüchtiges Öl mit einem Molekulargewicht größer als 650 g/mol umfasst.

10. Zusammensetzung nach einem der vorhergehenden Ansprüche, **dadurch gekennzeichnet, dass** sie weniger als 5 Gew.-% Wasser umfasst.

11. Zusammensetzung nach einem der vorhergehenden Ansprüche, **dadurch gekennzeichnet, dass** ihr mittleres Elastizitätsmodul G' zwischen 0,1 und 120.000 Pa, bevorzugt zwischen 100 und 50.000 Pa, mehr bevorzugt zwischen 700 und 35.000 Pa beträgt.

12. Zusammensetzung nach einem der vorhergehenden Ansprüche, **dadurch gekennzeichnet, dass** ihr mittleres Viskositätsmodul G" zwischen 30 und 30.000 Pa, bevorzugt zwischen 100 und 10.000 Pa, mehr bevorzugt zwischen 300 und 5000 Pa beträgt.

13. Zusammensetzung nach einem der vorhergehenden Ansprüche, **dadurch gekennzeichnet, dass** sie in Form eines Lidschattens, eines Lipgloss, eines Lippenstifts, eines Eyeliners oder eines Gesichtspuders vorliegt.

## Revendications

1. Composition cosmétique pour le maquillage ou le soin de la peau comprenant, dans un milieu physiologiquement acceptable :
(a) 0,6 à 20% en poids par rapport au poids total de ladite composition d'au moins une cire polyester ayant un point de fusion compris entre 40 et 90°C, et dont le taux de cristallinité est inférieur à 95%, de préférence inférieur à 90% ou encore inférieur à 75% et supérieur 45%;
dans laquelle le taux de cristallinité de la cire polyester à 25°C est égal au ratio entre la quantité totale de cristaux présents à une température de 25°C ramené à 200 J/g,
ladite cire de polyester étant choisie parmi :
- un polyester d'au moins un polyol et d'au moins deux, voire d'au moins trois, acides monocarboxyliques aliphatiques, linéaires ou ramifiés, saturés ou insaturés, identiques ou différents, ayant indépendamment les uns des autres de 8 à 30 atomes de carbone,
- une cire polyester obtenue à partir (i) d'un acide monocarboxylique aliphatique, linéaire ou ramifié, saturé ou insaturé, ayant de 8 à 30 atomes de carbone, (ii) d'un diacide linéaire ou ramifié ayant de 12 à 36 atomes de carbone, et (iii) du glycérol,
- les dérivés de cires d'abeille choisis parmi les cires de nom INCI Synthetic Beeswax, Bis-PEG-12 Dimethicone Beeswax, et leurs mélanges,
- un polyester obtenu par réaction i) d'un dimère diacide de l'acide isostéarique avec ii) un alcool aliphatique,
et
(b) de 1 à 25% par rapport au poids total de ladite composition d'au moins un copolymère acrylate/polytriméthylsiloxyméthacrylate.

2. Composition cosmétique selon la revendication 1, **caractérisée en ce que** ladite cire et le dit copolymère sont dans une quantité telle que ladite composition présente à 20°C en atmosphère ambiante:
- un module élastique G' moyen compris entre 0,1 et 120 000 Pa, et
- un module visqueux G'' moyen compris entre 30 et 30 000 Pa.

3. Composition selon la revendication 1, **caractérisée en ce que** la cire polyester est obtenue à partir (i) dudit acide monocarboxylique aliphatique choisi parmi les acides laurique, myristique, palmitique, oléique, stéarique, 12-hydroxystéarique, béhénique et leurs mélanges, (ii) d'un diacide linéaire ou ramifié ayant de 12 à 36 atomes de carbone, et (iii) du glycérol.

4. Composition selon l'une quelconque des revendications précédentes, **caractérisée en ce que** la cire polyester est un diester d'acide eicosadioique et de glycérol estérifié par l'acide béhénique, et éventuellement par l'acide isostéarique.

5. Composition selon les revendications précédentes, **caractérisée en ce que** cire polyester est un ester du saccharose de l'acide stéarique et de l'acide acétique.

6. Composition selon l'une quelconque des revendications 1 à 5, **caractérisée en ce que** la cire polyester représente de 0,6 à 15% en poids, de préférence de 1 à 10% en poids, de préférence encore de 2 à 8 % en poids du poids de la composition.

7. Composition selon l'une quelconque des revendications précédentes, **caractérisée en ce que** le ratio massique entre le copolymère acrylate/polytriméthylsiloxy-méthacrylate et la cire polyester est compris entre 5/1 et 1/1.

8. Composition selon l'une quelconque des revendications précédentes, **caractérisée en ce qu'**elle comprend de 0,1 à 80% en poids par rapport au poids de la composition d'au moins une huile volatile, telle que l'isododécane ou la cyclopentadisiloxane.

9. Composition selon l'une quelconque des revendications précédentes, **caractérisée en ce qu'**elle comprend au moins une huile non volatile de masse moléculaire supérieure à 650 g/mol.

10. Composition selon l'une quelconque des revendications précédentes, **caractérisée en ce qu'**elle comprend moins de 5% en poids d'eau.

11. Composition selon l'une des revendications précédentes, **caractérisée en ce que** son module élastique G' moyen est compris entre 0,1 et 120 000 Pa, de préférence entre 100 et 50 000 Pa, de préférence encore entre 700 et 35 000 Pa.

12. Composition selon l'une des revendications précédentes, **caractérisée en ce que** son module viqueux G'' moyen est compris entre 30 et 30 000 Pa, de préférence entre 100 et 10 000 Pa, de préférence encore entre 300 et 5 000 Pa.

13. Composition selon l'une quelconque des revendications précédentes, **caractérisée en ce qu'**elle est sous la forme d'une ombre à paupières, d'un gloss à lèvres, d'un rouge à lèvres, d'un eye-liner ou d'un fard à joues.
